Europäisches Patentamt

European Patent Office

Office européen des brevets

(11) Publication number: **0 363 063**
**A2**

(12) **EUROPEAN PATENT APPLICATION**

(21) Application number: **89309778.2**

(22) Date of filing: **26.09.89**

(51) Int. Cl.⁵: **C12N 15/18 , C12P 21/02 , A61K 37/36 , A61K 9/22 , A23K 1/165**

(30) Priority: **29.09.88 AU 684/88**

(43) Date of publication of application:
**11.04.90 Bulletin 90/15**

(84) Designated Contracting States:
**AT BE CH DE ES FR GB GR IT LI LU NL SE**

(71) Applicant: **Bunge (Australia) Proprietary Limited**
**6th Floor 616 St. Kilda Road**
**Melbourne Victoria 3004(AU)**

(72) Inventor: **Brandon, Malcolm Roy**
**14, Castella Street**
**Ivanhoe East 3079, Victoria(AU)**
Inventor: **Adams, Timothy Edward**
**48 Brassey Avenue**
**Rosanna 3084, Victoria(AU)**

(74) Representative: **Harding, Richard Patrick et al**
**Arthur R. Davies & Co. 27 Imperial Square**
**Cheltenham GL50 1RQ(GB)**

(54) **Sheep growth hormone.**

(57) In order to produce relatively large quantities of recombinant polypeptides having good ovine growth hormone activity which can be used in vivo as effective substitutes for the natural hormone, a complementary DNA (cDNA) sequence coding for a recombinant polypeptide having ovine growth hormone activity is provided together with a suitable cloning vector, so that the DNA sequence and cloning vector are ligated to deploy the DNA sequence into the cloning vector to form a molecular clone. The vector so formed may include a complete or partial copy of the ovine growth hormone polypeptide coding region, encompassing the mature hormone, extending past the 3' termination codon. The DNA sequence coding may be derived from polyadenylated messenger RNA (mRNA) isolated from ovine pituitary glands by treating the mRNA with reverse transcriptase in the presence of oligodT primer to synthesize first strand complementary DNA (cDNA), enzymatically removing the mRNA with RNase H, and subsequently synthesising the second cDNA strand with DNA polymerase I, and treating the double stranded complementary DNA (cDNA) so formed with T4 DNA polymerase to create flush (blunt) ended molecules.

EP 0 363 063 A2

## Sheep Growth Hormone

The present invention relates to a recombinant ovine growth hormone, a process for the production of a recombinant ovine growth hormone gene, and to processes for the construction of plasmid expression vectors for the expression, and purification, of high levels of ovine growth hormone in prokaryotic organisms.

Growth hormone, a polypeptide hormone, is synthesized in the anterior lobe of the pituitary gland as a large precursor molecule. Enzymatically the signal peptide is cleaved from the precursor, to yield the biologically active, mature form of growth hormone. Growth hormone, well recognized as a general anabolic agent, promotes a myriad of physiological effects in mammals and other vertebrates. It is a growth promoter responsible for skeletal growth, and a potentiator of protein synthesis. Growth hormone also displays insulin-potentiating properties, has weak lactogenic activity, and plays a role in lipid metabolism and homeostatic maintainence. There is some degree of species-specificity with respect to growth hormone; for example bovine growth hormone is inactive in man or monkey, but will elicit effects in rats and goats.

With respect to the ovine industry, administration of growth hormone promotes weight gain and improves carcass quality, thereby enhancing the feed conversion ratio. This is illustrated for example in copending Australian Patent Application 62522/86 the entire disclosure of which is incorporated herein by reference. However, while an economically viable role for growth hormone administration may be relevant with respect to the ovine industry, the supply of available natural, pituitary-derived ovine growth hormone would fall well short of demand. Given the species specificity of the product, sources of other naturally occurring growth-promoting polypeptides cannot be used. However, the developments of recombinant DNA technology, and subsequent applications thereof, allows for the manipulation of ovine growth hormone-encoding DNA sequences, allowing for the production of high levels of this protein in a prokaryotic organism, such as bacteria.

Accordingly, it is an object of the present invention to overcome, or at least alleviate, one or more of the difficulties related to the prior art. In particular we have been able to produce, using the processes described hereinafter, relatively large quantities of recombinant polypeptides having good ovine growth hormone activity. The polypeptides have good biological activity and can be used in vivo as effective substitutes for the natural hormone.

Accordingly, in a first aspect according to the present invention, there is provided a process for the preparation of a molecular clone which process includes
providing
a complementary DNA (cDNA) sequence coding for a recombinant polypeptide having ovine growth hormone activity; and
a suitable cloning vector;
ligating the DNA sequence and cloning vector to deploy the DNA sequence into the cloning vector to form a molecular clone.

The vector so formed may include a complete or partial copy of the ovine growth hormone polypeptide coding region, encompassing the mature hormone, extending past the 3′ termination codon.

The DNA sequence coding for a polypeptide having ovine growth hormone activity may be derived from polyadenylated messenger RNA isolated from ovine pituitary glands.

Accordingly, in a further aspect of the invention, there is provided a process for the preparation of a DNA sequence coding for a polypeptide having ovine growth hormone activity, which process includes the preliminary step of
providing a source of ovine pituitary gland;
isolating polyadenylated messenger RNA (mRNA) therefrom;
treating the mRNA with reverse transcriptase in the presence of oligodT primer to synthesize first strand complementary DNA (cDNA);
enzymatically removing the mRNA with RNase H, and subsequently synthesising the second cDNA strand with DNA polymerase 1; and
treating the double stranded complementary DNA (cDNA) so formed with T4 DNA polymerase to create flush (blunt) ended molecules.

The polyadenylated RNA isolation step from ovine pituitary gland may be achieved in any suitable manner, for example, by utilising a guanidine thiocyanate treatment and affinity chromatography.

The suitable cloning vector according to the present invention may be selected from a variety of prokaryotic cloning vectors. The bacteriophage virus vector λgt10 and the like have been found to be suitable.

The ligation step may take any suitable form. The cloning reaction step maybe by means of ligation by the addition of synthetic DNA linkers. In this case linkers corresponding to the Eco RI restriction enzyme recognition sequence may be attached, utilizing T4 DNA ligase, to either end of the blunt ended cDNA molecules.

Digestion of the aforementioned cDNA with the enzyme Eco RI will thus yield cDNA molecules with 5' Eco RI cohesive overhanging termini.

Such molecules may then be cloned into the compatible Eco RI DNA site in gt10 by ligation; such DNA may then be packaged into virus particles "in vitro" and subsequently used to infect appropriate bacterial microorganisms to create a recombinant cDNA library from which recombinant clones containing cDNA sequences encoding ovine GH may be selected.

Accordingly, in a preferred aspect of the present invention the molecular clone may be a bacteriophage clone selected from the clones designated OGH1-12 and OGH1-14 as hereinafter described .

Samples of the bacteriophage clones are maintained in the collection maintained by Bunge (Australia) Pty. Ltd. North Melbourne, Victoria, Australia.

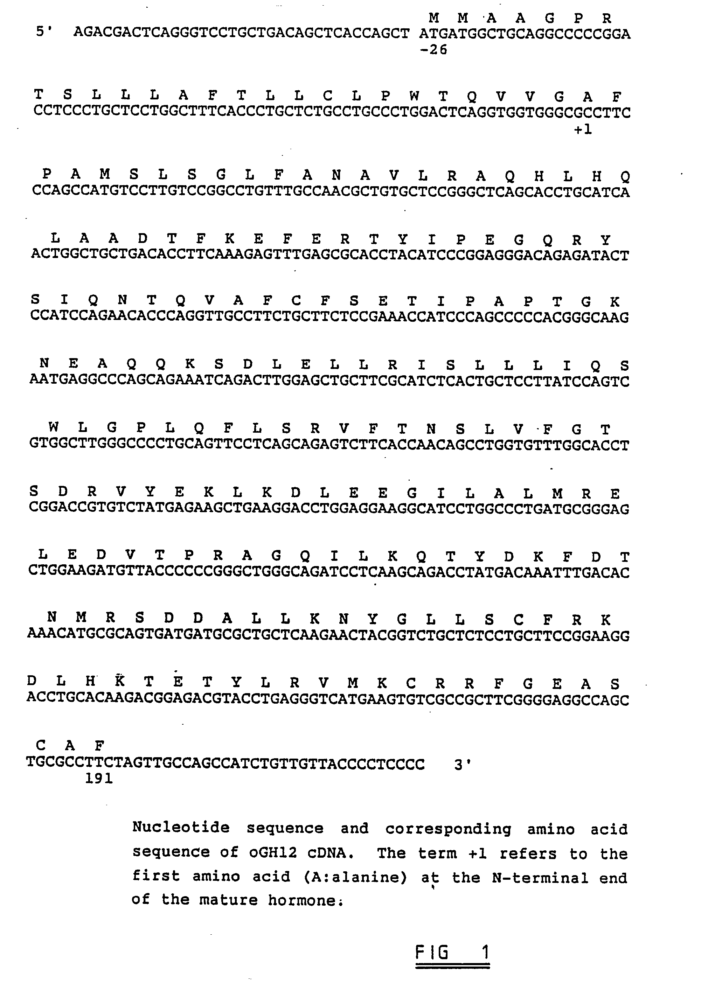

The bacteriophage clone OGH12 is preferred. The nominated clone contains a complete copy of the ovine growth hormone polypeptide coding region, including 5' and 3' untranslated flanking sequences. The entire DNA sequence of OGH1-12 was established and is presented in Figure. 1.

It should be understood that while such bacteriophage vectors provide a useful vehicle for the stable maintainence of the DNA sequence coding for a polypeptide having ovine growth hormone activity, such vectors do not represent the vehicle of choice for the expression of eukaryotic genes in prokaryotic cells. Expression of eukaryotic genes in prokaryotic cells require expression vectors that utilise defined regulatory sequences controlling transcription and translation of the foreign gene, in order to achieve the proper expression of that gene in prokaryotic cells transformed with that vector.

Thus, in a still further aspect of the present invention, there is provided a process of preparing a plasmid having a sequence coding for a recombinant polypeptide having ovine growth hormone activity and capable of being replicated, transcribed and translated in a unicellular organism which process includes providing

a restricted plasmid expression vector having excluded therefrom a DNA sequence of predetermined length; and

a DNA sequence coding for a recombinant polypeptide having ovine growth hormone activity, or a portion thereof, and including a synthetic end sequence satisfying the regulatory requirements for transcription and translation in a unicellular organism; and

ligating the DNA sequence to the restricted plasmid expression vector.

The restricted plasmid expression vector according to this aspect of the present invention may be of any suitable type. A dual-origin vector is preferred. A dual-origin vector is one in which one origin of replication is responsible for the stable maintenance of plasmid at low copy number whilst the other, switched on by a change in temperature, directs constitutive expression of the cloned gene and enhanced copy number. Thus, induction of copy number amplification and gene expression in large scale cultures is relatively cheap and simple.

A restricted plasmid expression vector may be formed from the plasmid pMG197, a derivative of pMG411 (described in Gene, 1984 by Yarrington et al). pMG197 is a dual origin plasmid expressing met-Gastric Lipase enzyme from the trp promoter which has a Shine-Dalgarno (SD) sequence 14 base pairs upsteam from the AUG start codon.

pMG197 may be modified to form a restricted plasmid expression vector. pMG197 may be modified by removing the EcoRI site between the par locus and the c1857 sequences and by excluding the met-gastric lipase gene as a Bgl II-Eco RI fragment.

In order to achieve the appropriate construction a process involving multiple steps is required. This necessitates:

(i) the synthesis of synthetic oligonucleotides corresponding to the 5' coding sequence of the mature ovine GH polypeptide to facilitate cloning into the Bgl II site of restricted pMG197. Furthermore, the codons selected for the synthesis of the oligonucleotides, while satisfying the relevant ovine GH amino acids, should represent those corresponding to the most abundant t RNAs found in E. coli.

Accordingly in a preferred embodiment of this aspect of the invention, wherein the DNA sequence coding for a recombinant polypeptide includes a portion only of the DNA sequence coding for ovine growth hormone, consisting of a synthetic 5' end sequence and a 3' end sequence, said method further including providing a further DNA sequence including the remainder of the complete DNA sequence coding for ovine growth hormone;

cleaving the said DNA sequence at a restriction site between the synthetic 5' end sequence and the 3' end sequence; and
ligating the further DNA sequence into the restriction site.

Preferably one of the two general types of oligonucleotides may be used as the synthetic 5' end sequence of the said DNA sequence. Thus two general types of oligonucleotides may be designed to replace the DNA coding for the first 14 amino acids of the OGH coding sequence.

The 5' translated region of the oGH cDNA contains the eukaryotic signal sequence for transport of the hormone across the endoplasmic reticulum in the eukaryotic cell. The sequence coding for the mature protein starts 79 base pairs downstream of the first nucleotide utilized in the signal peptide, so that in the prokaryote expression system the eukaryotic signal peptide must be deleted. The 5' terminus of the oGH cDNA coding for the mature protein starts with the codon for alanine, GCC. Bacterial expression, however, requires the codon, ATG, for initiation. This codes for methionine, so that the mature protein resulting from bacterial expression will begin with a methionine rather than an alanine. The effect of making this modification was not known, and therefore two alternative oligonucleotide pairs were prepared to modify the 5' terminus; each providing an ATG initiation codon. One oligonucleotide pair coded for a methionine in front of the alanine found naturally at the NH2 terminus of the mature protein. The other omitted the four NH2 terminal amino acids and therefore started with amino acid number 5, the first naturally occurring methionine. These were designated, met:1-15 and met:5-15, respeotively, coding for oGH amino acids 1-15 and 5-15. Accordingly, in a further aspect of the present invention there is provided an oligonucleotide sequence selected from oligonucleotide Met:1-15 and Met5-15

The first type of oligonucleotide, oligonucleotide Met:1-15, includes a 5' Bgl II cohesive end, an initiation codon (ATG) corresponding to an NH₂-terminal methionine residue not found in the mature ovine growth hormone polypeptide, codons corresponding to amino acids 1-14 of the natural hormone (mature form) and terminating in a 3' overhang that corresponds to a HgiA I site found at nucleotide 160. Met:1-15 has the structure as follows:

```
5' GATCTATGGCTTTTCCAGCTATGTCTCTTTCTGGTCTGTTCGCTAACGCTGTGCT 3'

55mer

3'      ATACCGAAAAGGTCGATACAGAGAAAGACCAGACAAGCGATTGCGAC       5'

47mer
```

The second oligonucleotide ,oligonucleotide Met: 5-15 has both 5' Bgl II and 3' HgiA I cohesive termini but initiates at a codon corresponding to a naturally occurring methionine residue (amino acid 5, mature hormone) followed by codons representing amino acids 6-14. Met:5-15 has the structure as follows:

```
5' GATCTATGTCTCTTTCTGGTCTGTTCGCTAACGCTGTGCT                      3'

40mer

3'      ATACAGAGAAAGACCAGACAAGCGATTGCGAC                          5'

32mer
```

In addition to providing an ATG codon, the oligonucleotide sequences also had a BgIIIsite at the 5' end for cloning into the corresponding site in the vector. Furthermore, when designing the oligos, codons were selected, that whilst conserving oGH amino acid sequence, corresponded to the most abundant tRNA's found in E.coli. A further consideration was to minimise the use of G-C base pairs and substitute A-T base pairs were possible, to remove potential secondary structure within the ribosome binding site of the messenger RNA, which may otherwise adversely affect transcription.

Both oligos terminated at the 3' end with an HgiAl 3' overhang to enable each to be ligated to the remainder of the oGH cDNA via this site.

Various regulatory elements are involved in bacterial expression and must be present in the final construct. They may influence transcription, as in the promoter region, or translation, requiring a Shine-Dalgarno (SD) site and ATG initiation codon. The initiation codon, AUG on the mRNA, is responsible for initiation by pairing with f-met tRNA and thus facilitating translation. The efficiency of translation of an mRNA in bacteria is critically dependent on the presence of a ribosome binding site, the SD sequence, a group of six to eight purine nucleotides; and on the distance between this site and the AUG initiation codon. For efficient expression of eukaryotic proteins using expression vectors, the SD sequence is usually included in the vector itself. Since the distance between the AUG and this site is important, the vector has been designed such that when the 5' end of the cDNA is ligated via the BglII site, the ATG, when immediately downstream of this restriction sequence, is at a distance of 14 bp from the SD sequence. This was found empirically, to be optimal for various eukaryotic proteins expressed from the dual origin vector.

The 3' untranslated end of the oGH cDNA contained the polyA signal sequence and about 200bp of adenine nucleotides, before the EcoRI site. Immediately downstream of the coding region, i.e. next to the last coding triplet is a termination codon, TAG, and another one a further 40bp downstream, before the polyA signal.

Thus according to a further aspect of the invention there is provided a process for the production of a recombinant polypeptide having ovine growth hormone activity which process includes the steps of providing

a recombinant plasmid expression vector including a DNA sequence coding for a polypeptide having ovine growth hormone activity and capable of being replicated, transcribed and translated in a unicellular organism, and

a unicellular organism;

introducing said recombinant plasmid expression vector into said unicellular organism by a method selected from transformation, transduction, or transfection;

culturing the resulting organism;

expressing the recombinant polypeptide encoded by said DNA sequence; and optionally

isolating said polypeptide from the culture.

In the process according to the invention, the unicellular organism may be a prokaryotic organism, for example a bacterial strain such as a strain of E. coli. The E. coli DH1 and E. coli 1B392 have been found to be particularly suitable.

The recombinant plasmid expression vector for use in this aspect of the invention may be any suitable vector prepared using the techniques described above. Particularly preferred vectors include pMGoGH1and pMGoGH2.

The process according to the invention is particularly suitable for preparing recombinant polypeptides oGH1-191 and oGH5-191 as described herein.

Steps (1)-(4) of the process according to the invention may be carried out using well-known methodology, for example as described in the Examples hereinafter. Where it is desired to isolate the polypeptide product according to steps (5), conventional procedures may also be used. Thus, after cell disruption, e.g. by cell lysis, the isolation of the polypeptide may be conducted utilising for example chromatography involving ion exchange, affinity or sizing resins, and/or by sedimentation e.g. centrifugation or by other known techniques for the purification of polypeptides.

Where the recombinant polypeptide is expressed as an insoluble aggregate and/or denatured, solubilisation and/or renaturation may be effected using conventional techniques, for example as described in International Patent Specification WO 83/04418, UK Patent Specification 2138004 and European Patent Specification 226448.

Particularly useful organisms for use in step (3) of the process according to the invention include E. coli strains, especially E. coli 1B392 containing one of the recombinant plasmid expression vectors pMGoGH1 and pMGoGH2, and such strains, together with mutants, recombinants and genetically-engineered derivatives thereof form a further aspect of the invention.

The level of expression is directly related to plasmid copy number. Ovine growth hormone is constitutively expressed from the trp promoter such that the final amount of oGH produced from oGH cDNA is determined by the number of copies in the cell. There is a certain amount of "leakage" in uninduced cells, but this has not been shown to present a problem when growing cells up to the required uninduced biomass. An O.D. $=107$ prior to induction was obtained in the 200L fermentation.

Particularly useful recombinant polypeptides we have prepared using the process of the invention include the ovine growth hormone variants oGH1-191 and oGH5-191. Both recombinant polypeptides have surprisingly good in vivo activity relative to the naturally occurring hormone and may be used in animals, especially ovine and bovine, for example to improve carcass quality and/or to promote weight gain. For use in this way oGH1-191 and oGH5-191 may be administered to the animal in veterinary formulations.

Thus according to a further aspect of the invention we provide a veterinary composition including a recombinant polypeptide having ovine growth hormone activity selected from oGH1-191 or oGH5-191 and the veterinarily acceptable salts thereof together with one or more carriers acceptable for veterinary use.

5

The term oGH1-191 as used herein is intended to mean a recombinant polypeptide with the amino acid sequence of naturally occurring ovine growth hormone, an additional methionine residue at the N-terminus having been removed as described below. The term OGH5-191 is intended to mean a polypeptide with the amino acid sequence of naturally occurring ovine growth hormone except that the first four N-terminal amino acids (Ala-Phe-Pro-Ala) are not present.

Veterinarily acceptable salts of oGH1-191 and OGH5-191 include salts of acids or bases, for example inorganic acid salts such as hydrochlorides, or inorganic base salts such as alkali metal, e.g. sodium, salts.

The compositions according to the invention may take any form suitable for administration including forms suitable for oral, rectal or parental (including implant) use. For oral administration the compositions may take the form of, for example solutions, syrups or suspensions e.g. in aqueous buffer, or solid compositions such as tablets or capsules, prepared by conventional means. For parental use, the compositions may for example take a form suitable for injection, such as a suspension, solution or emulsion in an aqueous or oily vehicle optionally containing formulatory agents such as suspending, stabilising, solubilising and/or dispersing agents.

In the composition according to the invention the concentration of active ingredient may be varied for example depending on the nature of the animal to be treated and the desired effect, but in general sufficient will be used to facilitate the administration of a dose of active ingredient in the range 0.01 to 0.2mg/kg liveweight/day.

The compositions according to the invention may be prepared by conventional means and in a further aspect of the invention we provide a process for the manufacture of a veterinary composition comprising bringing into association an aliquot amount of a recombinant polypeptide having ovine growth hormone activity selected from oGH1-191 or OGH5-191 and the veterinary acceptable salts thereof with one or more carriers acceptable for veterinary use.

As stated above, we have found that supplementary growth hormone levels in sheep and cattle with recombinant ovine growth hormone result in improved carcass quality as assessed by back fat thickness, fat and lean percentage, or lean eye area, optionally together with improved feed conversion efficiency.

No deleterious effect upon the health of the animals was observed and this was confirmed by pathological examination of all the body tissues of the animals.

Accordingly, in a further preferred aspect of the present invention there is provided a method for the treatment of an animal to improve carcass quality and food conversion efficiency, which method includes providing
an animal selected from the group consisting of entire male, female and castrated male sheep and bovine animals;
an exogenous recombinant ovine growth hormone, analogues, derivatives, or fragments thereof; and administering to the animal at a preselected liveweight and at a preselected constant dose rate depending on the sex of the animal, said exogenous recombinant ovine growth hormone initiated at a liveweight of approximately 35 to 100 kg and when the animal is female or a castrated male, the dose rate is approximately 0.06 to 0.10 mg/kg liveweight/day; and when the animal is an entire male, the dose rate is approximately 0.01 to 0.15 mg/kg liveweight/day.

Preferably, the recombinant ovine growth hormone is selected from oGH1-191 and oGH5-191.

Preferably, the animal to be treated is a sheep.

In a preferred embodiment, the administration of recombinant growth hormone is initiated at a liveweight of approximately 15 to 50 kg.

Within the preferred initiation range the improvement in carcass quality is essentially similar to that achieved over the longer period. A greater relative improvement in feed conversion efficiency is noted.

Improvement in carcass quality can be measured as an overall increase in lean tissue content together with an increase in the proportion of lean tissue content may be attributed to an increased overall growth rate, an anabolic effect of a combination thereof.

The increase in the proportion of lean tissue to fat tissue may be attributed both to the anabolic effect and a decrease in fat tissue content.

Specifically, we have surprisingly found that there is a previously unsuspected critical interaction between dose of GH, the time of commencing treatment, the length of treatment, dietary energy supply, and sex of the animal. The improved carcass quality in female and castrated male sheep and cattle was particularly unexpected, since normally they lay down fat more readily than entire males and produce an inferior carcass.

Our studies have shown that treatment according to the present invention enhances the growth rate in treated animals relative to controls. Moreover, the carcass quality is greatly improved and the feed consumption of the animals is lower, as a result of the more efficient utilisation of feed by animals treated with GH. At supra-optimal doses of GH, growth rate is not further enhanced, as apparently an appetite-suppressing effect can become limiting.

In an alternative aspect of the present invention, there is provided a method of decreasing fat content of an animal carcass, which method includes:

providing
an animal selected from the group consisting of entire male, female and castrated male sheep and bovine animals,
an exogenous recombinant ovine growth hormone, analogues, derivatives or fragments thereof;
administering to the animal said exogenous recombinant ovine growth hormone at a generally constant dose rate; wherein when the animal is a female or castrated male, the dose rate is approximately 0.06 to 0.1 mg/kg liveweight/day and the treatment continues for approximately 25 days prior to slaughter; when the animal is an entire male, the dose rate is approximately 0.1 to 0.15 mg/kg liveweight/day and the treatment continues for approximately 20 days prior to slaughter.

In a particularly preferred embodiment, the treatment is administered in a slow release form, for example in the form of implantable pellets or injectable pellets or injectable emulsion.

Whilst the methods described above may improve the carcass quality characteristics greatly, the utilisation of recombinant ovine growth hormones is extremely expensive and time consuming since it normally requires daily administration of unit dosage amounts of synthetic porcine growth hormone for a period of approximately 30 days or more.

Accordingly, in a further aspect of the present invention, there is provided a method for the treatment of an animal to improve carcass quality and/or food conversion efficiency, which method includes
providing
an animal selected from the group consisting of entire male, female and castrated male sheep and bovine animals;
an exogenous recombinant ovine growth hormone, analogues, derivatives, or fragments thereof; and
administering to the animal at a preselected liveweight and at a preselected generally constant dose, depending on the sex of the animal, said exogenous recombinant ovine growth hormone, at intervals of at least one day.

It has been surprisingly found that altering the periodicy of dosing provides a significant improvement in growth performance compared to controls. Whilst we do not wish to be restricted by theory, it is postulated that the modification of periodic of dosing leads to alteration of the relative, anit-lipogenic and protein stimulatory effects of recombinant growth hormone on adipose and muscle tissue respectively. It is further postulated that high doses of synthetic growth hormone given daily depress lipogenesis and feed intake to such an extent that the associated improvement in growth rate is generally only small and often undetectable.

By altering periodicy to every second, third or fourth day administration, that is at intervals of 1, 2 or 3 days, it is postulated that the protein stimulatory effect, which is mediated via another hormone (insulin-like growth factor 1), is maintained but the antilipogenic effect which is direct is reduced.

More preferably, the dose is approximately 10 mg of exogenous recombinant ovine growth hormone administered on every second, third or fourth day for a period of approximately 10 to 30 days, more preferably to 30 days.

Administration of a dose of approximately 10 mg every second day is particularly preferred to maximize growth rate. Administration every fourth day provides a significant improvement in growth rate compared with controls but not the same magnitude as administration more frequently. However compared with controls food conversion efficiency is also improved by a dosage regimen of every fourth day.

In a further preferred aspect of the present invention the preselected generally constant dose used may be a reduced dose relative to that normally used in the prior art.

For example, where a dose rate of approximately 10 mg per day may be standard throughout the industry, the dose may be reduced to approximately 50% or less of the standard dosage. It has surprisingly been found that such a reduction in dose amount has a similar and even enhanced effect on growth rate compared to controls at the high amounts described above. In addition feed conversion efficiency may be significantly improved.

Accordingly, the combination of alteration of dose and periodicy of dosage may be used to alter the relative effects of synthetic growth hormone on growth rate, feed conversion efficiency and carcass quality (fat levels etc.). Thus it is possible to tailor the treatments to different types (genotype strains and sexes) of animals including sheep and cattle.

Accordingly, in a further preferred aspect of the present invention there is provided a method of further increasing the growth performance of an animal to be treated which method includes

EP 0 363 063 A2

subsequently continuing administering the exogenous recombinant ovine growth hormone at an increased dose daily for a further preselected period.

In a particularly preferred form, the exogenous recombinant ovine growth hormone is administered to the animal initially at a dose of approximately 4 to 8 mg at intervals of 1, 2, or 3 days for a period of approximately 10 to 25 days; and

subsequently continuing administration at an increased dose of approximately 6 to 10 mg daily for a further period of approximately 5 to 15 days.

It has surprisingly been found that the dosage regimen described above may lead to a further significant improvement in growth rate and food conversion efficiency over the total growth period whilst the later period of daily administration of synthetic growth hormone also leads to a further significant reduction in fat levels thus providing major improvements in carcass quality prior to slaughter.

Preferably the initial period of treatment may extend for approximately 10 to 25 days, more preferably 15 to 20 days. The later period of full dose administration may extend for a further period of approximately 5 to 15 days, more preferably 10 days.

The methods of treatment described above may be provided utilising a veterinary composition including an aqueous buffer solution of approximately 3.3 to 6.6 mg/ml of a source of recombinant ovine growth hormone, analogues, derivatives of salts thereof.

In a preferred aspect of the present invention, the recombiant ovine growth hormone may be provided in a sustained-release form. Any suitable delivery system may be used which will provide sustained release. Accordingly, the present invention provides a sustained release veterinary article including

an at least partially soluble carrier,

a plurality of at least partially soluble microcapsules embedded therein, and

an effective amount of synthetic growth hormone or an analogue, derivative, fragment or salt thereof, within the microcapsule.

The at least partially soluble carrier of the sustained release veterinary article may be a polymeric article. The polymeric article may take the form of an implant or bolus.

A polymer which will function as an adjuvant for the synthetic growth hormone may be used. A water-soluble polymer may be used. The polymer article may preferably degrade in approximately 8 to 24 hours after entering the body of the animal. A polymer of the polyvinyl pyrrolidone type may be used. A polyvinyl pyrrolidone polymer or copolymer may be used. The polymer should be selected to provide sufficient impact strength to withstand the impact of the selected delivery system. Other standard compounding ingredients may be incorporated into the polymer matrix. Such compounding ingredients may include fillers and extenders. The polymer matrix may further include other active ingredients. Antibiotics, dietary supplements, drenches and the like may also be included.

The polymeric article may be formed in any suitable manner. The polymeric article may be formed utilising an injection molding technique.

As stated above, the sustained-release implant according to this aspect of the present invention further includes a plurality of at least partially soluble microcapsules embedded in the carrier.

The plurality of microcapsules may be incorporated into the polymeric article during the polymerization step thereof. Alternatively, the microcapsules may be incorporated at the molding stage. For example the polymeric article may be compressed into a desired shape utilising tableting technology. A tablet press may be used. The microcapsules and polymer may be mixed prior to moulding.

The microcapsules may be formed from any suitable at least partially soluble polymeric material. A polyester polymer may be used. Polymers and copolymers of -hydroxy acids and derivatives thereof are preferred.

In a preferred aspect, the microcapsules may be formed from a first polymer or copolymer of glycolic acid, lactic acid, a derivative thereof or mixtures thereof having a relatively low molecular weight and a second polymer or copolymer of glycolic acid, lactic acid, a derivative thereof, or mixtures thereof having a relatively high molecular weight. More preferably the plurality of biodegradable microcapsules are formed in at least two particle sizes.

In a further preferred aspect, the plurality of biodegradable microcapsules include microcapsules having a relatively short degradation rate, a medium-term degradation rate or a relatively long degradation rate or a mixture thereof. As discussed above, degradation rates, and in turn the rate of release of the physiologically active ingredients incorporated therein, may be modified by utilising differing polymeric compositions and/or by modifying the molecular weight of the polymers used.

The recombinant ovine growth hormone may be encapsulated within the microcapsules in any suitable manner. The encapsulation process may include mixing the polyester or copolyester with the recombinant ovine growth hormone in a suitable solvent; and causing the polyester the precipitate.

8

Thus for example the recombinant polypeptide may be mixed or blended with, or suspended or dissolved in, a carrier as appropriate, using conventional techniques.

The present invention will now be more fully described with reference to the accompanying examples. It should be understood, however, that the following description is illustrative only and should not be taken in any way as a restriction on the generality of the invention described above.

## EXAMPLE 1

### Methods and Results

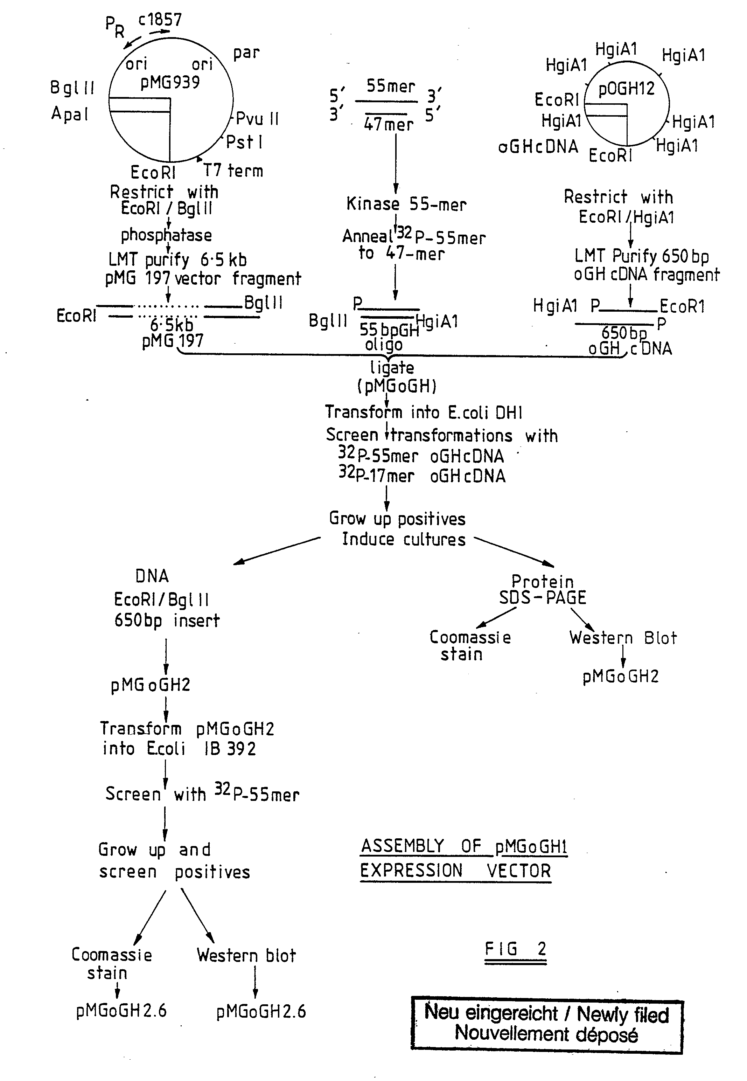

The method of construction of plasmid pMGoGH1 is schematically presented in Figure 2.

### (i) Preparation of expression vector pMG197

The final expression construct comprises a suitable vector for stably maintaining the cDNA in the host, and capable of efficient transcription and translation. These functions are provided by the plasmid expression vector pMG197. pMG939, a derivative of pMG197 encoding the expression of porcine growth hormone (PGH) from Celltech Limited, was restricted with EcoRI and BglII in medium salt restriction buffer, 50mM NaCl, 10mM Tris. Cl pH 7.5, 10mM MgCl2, 1mM dithiothreitol (DTT). It was then phosphatase treated with calf intestine alkaline phosphatase (CIP). Removal of the phospate groups minimises religation of the vector with itself. The main vector fragment was purified away from pGH cDNA by 0.8% low melting temperature (LMT) agarose gel electrophoresis. A slice of agarose containing the 6.5 kilobase pair (kb) vector fragment was cut from the gel and the DNA eluted by melting at 50°C and extracting with phenol in dry ice. The restriction vector pMG197, contained in the aqueous phase, was cleaned using QIAGEN.

### (ii) Preparation of oligomers for reconstructing 5′ terminus of the oGH gene coding for mature oGH protein

The two different pairs of oligonucleotides designed to be ligated to the 5′ HgiA1 site were synthesised by phosphotriester chemistry at Bresatec Ltd., Adelaide, South Australia.

5' GATCTATGGCTTTTCCAGCTATGTCTCTTTCTGGTCTGTTCGCTAACGCTGTGCT 3'

55mer

3'      ATACCGAAAAGGTCGATACAGAGAAAGACCAGACAAGCGATTGCGAC      5'

47mer

5' GATCTATGTCTCTTTCTGGTCTGTTCGCTAACGCTGTGCT      3'

40mer

3'      ATACAGAGAAAGACCAGACAAGCGATTGCGAC      5'

32mer

Met1-15 comprised the 55mer and the 47mer and was constructed as follows. To maximise efficiency of a three way ligation, the 55mer oligo was kinased before being annealed to the complementary 47mer. Although it was still possible for the oligos to form dimers via the BglII site, the pattern of phosphorylation of the components of the triple ligation increased the chances of them assembling in the correct order. The 55mer was kinased for 2 hours at 37°C in 10mM Tris.Cl pH7.5, 10mM MgCl2, 10mM DTT, 1mM ATP using 10 units of T4 polynu7cleotide kinase, and annealed to the 47mer by incubating at 65°C for 10 minutes and allowing to cool slowly to room temperature. The annealed oligos were ammonium acetate/ethanol precipitated to remove unincorporated 32-P and resuspended in 10mM Tris.Cl pHB, 1mM EDTA, (TE), ready for ligating. Similarly, the Met:5-15 oligonucleotide dimer was prepared by first kinasing the 40 mer oligonucleotide, followed by annealing to the 30 mer oligonucleotide.

(iii) Preparation of oGH cDNA

pOGH12 was restricted with HgiAI and EcoRI (in 150mM NaCl, 10mM Tris.Cl pH8.0, 10mM MgCl₂, 10mM mercaptoethanol, 100ug/ml BSA) to release a 650bp fragment corresponding or amino acids 16-191. This was purified by LMT 1% agarose electrophoresis and an agarose slice containing the 650 basepair (bp) fragment cut from the gel and melted for 10 minutes at 60°C. This was extracted with phenol in dry ice, and cleaned up using QAIGEN.

(iv) Ligation of EcoRI/BglII restricted vector, annealed oligonucleotides and HgiAI/EcoRI restricted fragment of oGH cDNA.

The ligation was carried out in 10mM Tris.Cl pH7.5, 10mM MgCl₂, 50mM NaCl, 10mM DTT, 1mM ATP overnight at 12°C using approximately 10 fold excess molarity of ends of oGH cDNA and 5' oligonucleotide dimer over vector, in the presence of 5U T4 DNA ligase. An example, using Met:1-15, of the ligation reaction is presented below:

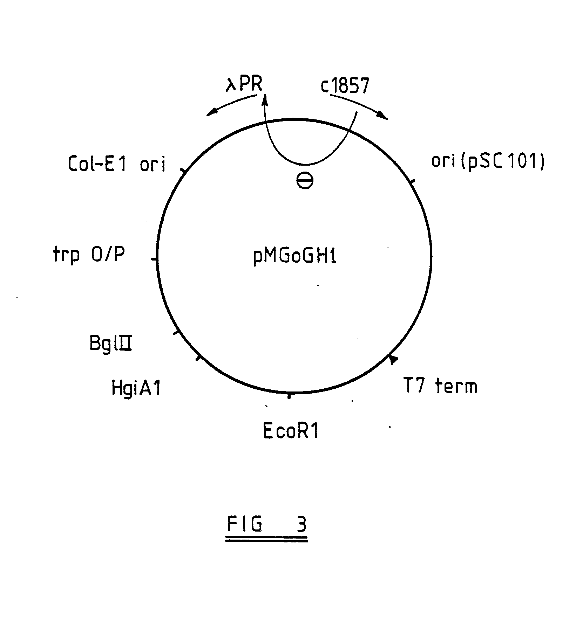

A resriction map of plasmid pMGoGH1 is given in Figure 3.

(v) Transformation of ligation products into competent E.coli DH1

Competent E.coli DH1 cells were made by the following method. Cells were cultured in Luria broth (LB) to an OD of 0.3 and harvested at 3000g. The cell pellet was resuspended in 0.4 volumes of chilled 50mM CaCl₂, and left on ice for 40 minutes. They were then spun down at 150g, and resuspended in 0.04 volumes of 50mM CaCl₂, and stored on ice for 40 minutes before being transformed with pMGoGH₂ ligation products.

The ligation was gently added to the competent E.coli DH1, left on ice for 20 minutes and then heat shocked at 42°C for 90 seconds. The cells were grown in Luria broth (LB) for 45 minutes to allow expresssion of the antibiotic resistance marker, and plated out on LB plates under Ampicillin (Amp) selection. The plates were grown overnight at 30°C.

(vi) Oligonucleotide screening of bacterial transformants

The transformants were replica plated such that uninduced and induced transformants could be screened and compared. A lift was taken from the master plate onto an Amersham Hybond filter and orientated with respect to the plate. A second filter was placed on top of the first, and both placed between two glass plates. The second filter was orientated with respect to the first. The filters were put onto fresh LB Amp plates, colony side up, and incubated at 30°C until bacterial colonies had regrown. One set of filters were then incubated at 42°C for 30 minutes, and then transferred to 37°C for 3 hours. This process constituted induction. Both sets of filters were then treated identically.

The filters were prewashed in 50mM Tris.Cl pH8.0, 1M NaCl, 1mM EDTA, 0.1% SDS, at 42°C for 1 hour. They were prehybridised in a solution containing 5 x SSPE (0.75M NaCl, 0.05M Na H₂ PO4. H₂O, 0.005M EDTA, pH 7.4), 5 x Denhardts solution (0.5% polyvinylpyrrolidine, 0.5% bovine serum albumin, 0.5% Ficoll 400), 0.5% SDS, 20 ug/ml denatured salmon sperm DNA, at 65°C for 4 hours. Radiolabelled probes were made by kinasing both the 55mer oligonucleotide and a 17mer (see below), with gamma ³²P-labelled ATP (Amersham Pty. Ltd.) as described by Maniatis et al. in "Molecular Cloning. A laboratory Manual." Cold Spring Harbor Laboratory, New York. The filters were first screened with the 32-P 55mer in the above prehybridisation solution at 65°C overnight. The other probe was stored at 4°C until required. 5' GATTTCTGGATGGAGTA 3'    oGH 17-mer

The filters were washed four times in 0.1x SSC/0.1% SDS at 65°C, and exposed to X-ray film overnight. The filters were boiled 30 minutes in distilled water to strip the previous probe, and rehybridised with the oGH 32P 17-mer at 65°C overnight. The filters were washed four times in 5x SSC/0.1% SDS; twice at 45°C and twice at 50°C. They were exposed overnight.

(vii) Growth of cultures of recombinant E.coli DH1 detected bv screening with radiolabelled oligonucleotides

Six colonies that gave positive signals with both probes were picked into 10ml LB Amp, and grown at 30°C. These cultures were used to inoculate 200 ml LB Amp cultures and lay down glycerol stocks, stored at -70°C. The large cultures were grown at 30°C until mid log phase (OD =0.5) and samples taken for DNA and protein analysis. They were then induced at 42°C for 30 minutes before being grown at 37°C for 4 hours. Samples were taken again.

(viii) Isolation and Purification of recombinant DNA from uninduced and induced E.coli DH1 transformant cultures

Recombinant DNA was isolated from uninduced and induced E.coli DH1 cultures by the SDS-alkaline lysis method. In this method, the cells are lysed, and the DNA denatured in 1% SDS/0.2M NaOH. Addition of 5M potassium acetate allows the small plasmid DNA to renature, but chromosomal DNA is too large to completely renature under these circumstances. A clearing spin pellets cellular debris and leaves the soluble nucleic acids in solution. These are isopropanol precipitated and DNA is purified away from RNA on a CsCl gradient. Induction was demonstrated as a higher DNA yield, by agarose gel electrophoresis.

Restriction of the purified plasmids with EcoRI and BglII, verified that five of the clones contained the 650bp OGH insert.

(ix) SDS-PAGE of crude DH1/pMGoGH cell extract

Cell pellets from both uninduced and induced cultures were resuspended in electrophoresis sample buffer, and samples run on two identical 12% SDS-polyacrylamide gels. One was Coomassie stained and the other analysed by Western blot analysis (see below) for the presence of immunoreactive growth hormone. The stained gel showed no significant induction of protein co-migrating with purified oGH.

(x) Western blot analysis of SDS-PAGE of DH1/pMGoGH

The electrophoresed proteins were electroblotted onto nitrocellulose and treated as follows. The filter was blocked in phosphate buffered saline (PBS)/1%tween/0.5%BSA, then incubated with a biotinylated monoclonal antibody to pGH, 21-51, in PBS/Tween/BSA. This was then reacted via the biotin moiety, with avidin conjugated to horseradish peroxidase in PBS/Tween/BSA. The blot was developed with diaminobenzide and hydrogen peroxide. A number of bacterial transformants, derived from both the Met:1-15 and 5-15 ligation reactions, bound the monoclonal antibody, and were chosen to be transformed into E.coli IB392 for further expression work.

(xi) Sequencing of pMGoGH clones to confirm reading frame

CsCl purified pMGoGH plasmid DNA was sequenced by the Sanger method modified for use with SEQUENASE (U.S. Biochemicals), according to the manufacturers instructions, and plasmid template. The 40mer, 32mer and l7mer were used as primers to sequence through the BglII and HgiAI sites and confirm reading frame.

11

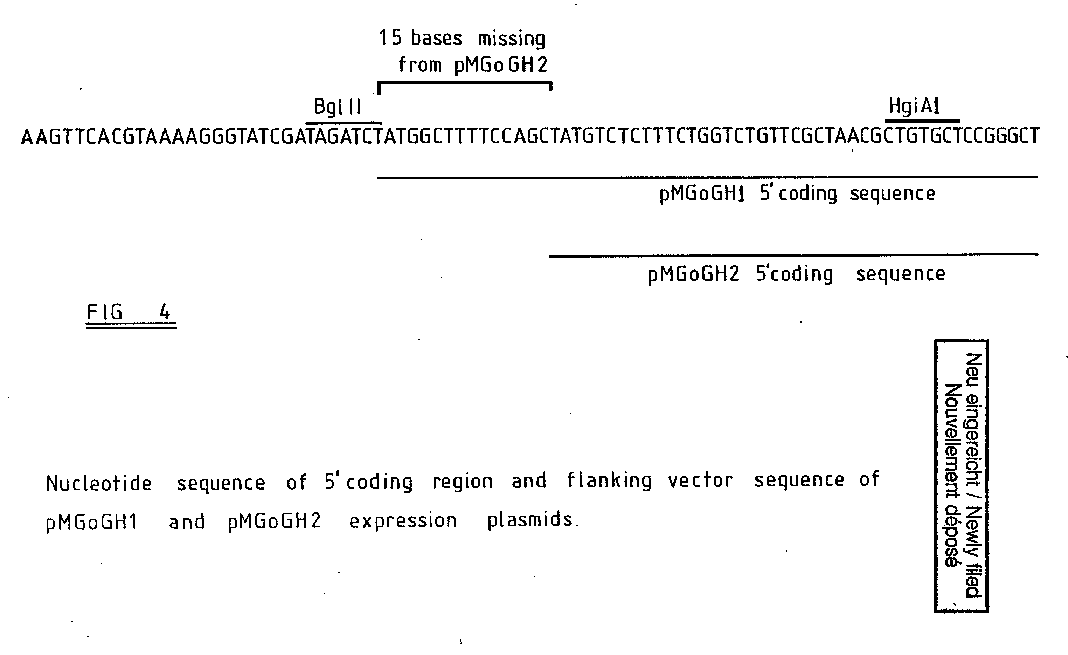

pMGoGH plasmid DNA was denatured in 0.2M NaOH for 5 minutes at room temperature and precipitated in 1.5M ammonium acetate/ethanol. 3ug of plasmid DNA to 0.5 pmoles oligonucleotide primer were annealed at 37°C for 20 minutes. The 35-S radiolabelling reactions were done at room temperature for 5 minutes, and terminated at 42°C for 5 minutes. Samples were denatured at 70°C for 10 minutes and loaded onto a 5% acrylamide sequencing gel. The gel was fixed in 10% acetic acid/10% methanol for 30 minutes and dried down before exposing to film overnight. The sequences of plasmids pMGoGH1 and pMGoGH2 are given in Figure 4.

(xii) Transformation of E.coli IB392 and expression of oGH

Competent E.coli IB392 cells were transformed with pMGoGH1 CsCl purified plasmid DNA. The cells were prepared by the PEG-divalent cation method. In this procedure cells are grown to an O.D. =0.3. They are then pelleted at 500g for 10 minutes and resuspended in 1/10 volume of chilled transformation and storage solution (TSS). TSS comprises LB containing 10%w/v PEG-8000, 5%v/v DMSO, 20mM $MgCl_2$, at pH6.5. 100 ul cells are incubated with the transforming plasmid on ice for 30 minutes. 0.9 ml TSS is added and the transforming cells are shaken at 37°C for 1 hour to allow expression of antibiotic resistance before plating on LB Amp (100 ug/ml) agar plates. The plates were incubated at 30°C overnight. Replica lifts of uninduced and induced colonies were made onto Hybond as described above, and screened with the 32-P radiolabelled 55-mer. Hybridisation and wash conditions were identical to those described for E. coli DH1 transformants.

All the colonies hybridised with the probe, and three were picked into 10ml LB Amp and grown up at 30°C overnight. These were used to inoculate large scale cultures and make glycerol stocks as previously described. DNA and protein analyses were carried out following the methods given for E.coli DH1.

Coomassie staining of protein from induced cultures showed a slight increase of a protein co-migrating with purified oGH with all three clones. Western blot analysis indicated a significant induction of a protein capable of binding the 21-51 antibody. DNA and protein data indicated bona fide expression of oGH1-191, pMGoGH1 was grown up in a 200L fermenter and oGH1-191 purified and solubilised at Bunge (Australia) Pty.Ltd.

## EXAMPLE 2

### Biological Activity

Ovine growth hormone derived from recombinant plasmids in E.coli was tested for biological activity. 100 ug/day of the protein were injected into 8 hypophysectomized rats/group over a four day period. Twenty-four hours after the last injection the rats were sacrificed and the right tibia isolated. The bone was cleaned and split at the proximal end in the midsagittal plane. The ipiphyseal cartilage can be distinguished from surrounding bone following staining with silver nitrate. The results are expressed as percentage increases:

|  | CONTROLS | RECOMBINANTS |
| --- | --- | --- |
|  | -ve | ol-191 |
| mean | 5.34 | 8.73 |
| % increase | 0 | 63% |
| -ve control : hypophysectomized rat + saline buffer | | |
| ol-191 : recombinant GH from pMGoGHI | | |

EXAMPLE 3

N-terminal amino acid sequence to recombinant oGH1-191

Recombinant oGH1-191, purified from induced pMGoGH⁺ IB392 bacteria using methodology that included a final step of reverse-phase high performance liquid chromatography (HPLC), was analysed by N-terminal amino acid sequencing using automated Edman degradation. The amino acid sequence for the first 30 amino acids of the recombinant protein are presented below:

```
1
Ala - Phe - Pro - Ala - Met - Ser - Leu -
                  10
Ser - Gly - Leu - Phe - Ala - Asn - Ala -
                              20
Val - Leu - Arg - Ala - Gln - His - Leu -
His - Gln - Leu - Ala - Ala - Asp - Thr -
      30
Phe - Lys
```

This sequence is identical to that found at the N-terminus of the mature ovine growth hormone derived from ovine pituitary glands (Fernandex et al., FEBS Letters 25:265-270). Surprisingly, the N-terminal methionine residue predicted is absent. It is postulated that the removal of the N-terminal methionine residue in the recombinant oGH1-191 is removed by the action of bacterial methionylaminopeptidase (MAP), which efficiently cleaves N-terminal methionines bonded to olanine residues (Sabin et al., Biotechnology 7:705-709, 1989). The recombinant hormone, therefore, is identical to the mature, pituitary-derived, ovine growth hormone.

Finally, it is to be understood that various other modifications and/or alterations may be made without departing from the spirit of the present invention as outlined herein.

## Claims

1. A process for the preparation of a molecular clone which process includes
providing
a complementary DNA (cDNA) sequence coding for a recombinant polypeptide having ovine growth hormone activity; and

a suitable cloning vector;

ligating the DNA sequence and cloning vector to deploy the DNA sequence into the cloning vector to form a molecular clone.

2. A process according to claim 1 wherein the complementary DNA sequence coding for a recombinant polypeptide includes a complete or partial copy of the ovine growth hormone polypeptide coding region encompassing the mature hormone, extending past the 3' termination codon.

3. A process according to claim 2 including the preliminary step of

providing a source of ovine pituitary gland;

isolating polyadenylated messenger RNA (mRNA) therefrom;

treating the mRNA with reverse transcriptase in the presence of oligodT primer to synthesize first strand complementary DNA (cDNA);

enzymatically removing the mRNA with RNase H, and subsequently synthesising the second cDNA strand with DNA polymerase I; and

treating the double stranded complementary DNA (cDNA) so formed with T4 DNA polymerase to create flush (blunt) ended molecules.

4. A process of preparing a plasmid having a sequence coding for a recombinant polypeptide having ovine growth hormone activity and capable of being replicated, transcribed and translated in a unicellular organism which process includes

providing

a restricted plasmid expression vector having excluded therefrom a DNA sequence of predetermined length; and

a DNA sequence coding for a recombinant polypeptide having ovine growth hormone activity, or a portion thereof, and including a synthetic 5' end sequence satisfying the regulatory requirements for transcription and translation in a unicellular organism; and

ligating the DNA sequence to the restricted plasmid expression vector.

5. A process according to claim 4 wherein the restricted plasmid expression vector is derived from the dual origin vector plasmid pMG197.

6. A process according to claim 5 wherein the DNA sequence coding for a recombinant polypeptide includes a portion only of the DNA sequence coding for ovine growth hormone, consisting of a synthetic 5' end sequence and a 3' end sequence, said method further including

providing a further DNA sequence including the remainder of the complete DNA sequence coding for ovine growth hormone;

cleaving the said DNA sequence at a restriction site between the synthetic 5' end sequence and the 3' end sequence; and

ligating the further DNA sequence into the restriction site.

7. A process according to claim 6 wherein the synthetic 5' end sequence is formed from a first oligonucleotide selected from oligonucleotide including a 5' Bgl II cohesive end, an initiation codon (ATG) corresponding to an NH2-terminal methionine residue not found in the mature ovine growth hormone polypeptide, codons corresponding to amino acids 1-15 of the natural hormone (mature form) and terminating in a 3' overhang that corresponds to a HgiA I site found at nucleotide 160; and

a second oligonucleotide having both 5' BglII and 3' HgiA I cohesive termini but initiates at a codon corresponding to a naturally occurring methionine residue (amino acid 5, mature hormone) followed by codons representing amino acids 6-15.

8. A plasmid expression vector including a DNA sequence coding for a polypeptide having ovine growth hormone activity selected from pMGoGH1 or pMGoGH2 as hereinbefore described.

9. A process for the production of a recombinant polypeptide having ovine growth hormone activity which process includes the steps of

providing

a recombinant plasmid expression vector including a DNA sequence coding for a polypeptide having ovine growth hormone activity and capable of being replicated, transcribed and translated in a unicellular organism, and

a unicellular organism;

introducing said recombinant plasmid expression vector into said unicellular organism by a method selected from transformation, transduction, or transfection;

culturing the resulting organism;

expressing the recombinant polypeptide encoded by said DNA sequence; and optionally

isolating said polypeptide from the culture.

10. A process according to claim 9 wherein the unicellular organism is a strain of E.coli selected from

E.coli DNl1 and E.coli 1B392.

11. A process according to claim 10 wherein the recombinant plasmid expression vector including a DNA sequence coding for a polypeptide having ovine growth hormone activity is pMGoGH1 or pMGoGH2.

12. A veterinary composition including recombinant polypeptide having ovine growth hormone activity selected from oGH1-191 or oGH5-191 and the veterinarily acceptable salts thereof together with one or more carriers acceptable for veterinary use.

13. A method for the treatment of an animal to improve carcass quality and food conversion efficiency, which method includes
providing
an animal selected from the group consisting of entire male, female and castrated male sheep and bovine animals;
an oxogenous recombinant ovine growth hormone, analogues, derivatives, or fragments thereof; and
administering to the animal at a preselected liveweight and at a preselected constant dose rate depending on the sex of the animal, said exogenous recombinant ovine growth hormone initiated at a liveweight of approximately 35 to 100 kg and when the animal is female or a castrated male, the dose rate is approximately 0.06 to 0.10 mg/kg liveweight/day; and when the animal is an entire male, the dose rate is approximately 0.01 to 0.15 mg/kg liveweight/day.

14. A method according to claim 13 wherein the exogenous recombinant ovine growth hormone is selected from oGH1-191 and oGH5-191.

15. A method according to claim 14 wherein the administration is initiated at a liveweight of approximately 15 to 50 kg.

16. A method of decreasing fat content of an animal carcass, which method includes
providing
an animal selected from the group consisting of entire male, female and castrated male sheep and bovine animals,
an exogenous recombinant ovine growth hormone, analogues, derivatives or fragments thereof, and
administering to the animal said exogenous recombinant ovine growth hormone at a generally constant dose rate; wherein when the animal is a female or castrated male, the dose rate is approximately 0.06 to 0.1 mg/kg liveweight/day and the treatment continues for approximately 25 days prior to slaughter; when the animal is an entire male, the dose rate is approximately 0.1 to 0.15 mg/kg liveweight/day and the treatment continues for approximately 20 days prior to slaughter.

17. A method according to claim 16 wherein the recombinant ovine growth hormone is selected from oGH1-191 and oGHS-191.

18. A method for the treatment of an animal to improve carcass quality and/or food conversion efficiency, which method includes
providing
an animal selected from the group consisting of entire male, female and castrated male sheep and bovine animals;
an exogenous recombinant ovine growth hormone, analogues, derivatives, or fragments thereof; and
administering to the animal at a preselected liveweight and at a preselected generally constant dose, depending on the sex of the animal, said exogenous recombinant ovine growth hormone, at intervals of at least one day.

19. A method according to claim 18, wherein the exogenous growth hormone is administered at intervals of 1, 2 or 3 days.

20. A method according to claim 19 wherein the recombinant ovine growth hormone is selected from oGH1-191 and oGH5-191.

21. A method according to claim 20, wherein the dose is approximately 10 mg of exogenous recombinant ovine growth hormone administered on every second, third or fourth day for a period of approximately 10 to 30 days.

22. A method for the treatment of an animal to improve carcass quality and/or food conversion efficiency, which method includes
providing
an animal selected from the group consisting of entire male, female and castrated male sheep and bovine animals;
an exogenous recombinant ovine growth hormone, analogues, derivatives, or fragments thereof; and
administering to the animal at a preselected liveweight said exogenous recombinant ovine growth hormone initially at a preselected generally constant reduced dose for a preselected period; and
subsequently continuing administering the exogenous recombinant ovine growth hormone at an increased dose daily for a further preselected period.

23. A method according to claim 22, wherein the exogenous recombinant ovine growth hormone is administered to the animal initially at a dose of approximately 4 to 8 mg at intervals of 1, 2 or 3 days for a period of approximately 10 to 25 days; and subsequently continuing administration at an increased dose of approximately 6 to 10 mg daily for a further period of approximately 5 to 15 days.

24. A method according to claim 23 wherein the exogenous synthetic growth hormone is administered to the animal initially at intervals of 1 or 2 days for approximately 20 days; and subsequently continuing administration daily for a period of approximately 10 days.

25. A sustained release veterinary article including
an at least partially soluble carrier;
a plurality of at least partially soluble microcapsules embedded therein; and
an effective amount of a recombinant ovine growth hormone or an analogue, derivative, fragment or salt thereof, within the microcapsules.

26. A sustained release veterinary article according to claim 25 wherein the article is an implant or bolus and the at least partially soluble carrier is a water soluble polymer which degrades in approximately 8 to 24 hours after entering the body of the animal.

27. A sustained release veterinary article according to claim 26 wherein the polymer is a polyvinyl pyrrolidone polymer or copolymer thereof.

```
                                                    M  M ·A  A  G  P  R
5'   AGACGACTCAGGGTCCTGCTGACAGCTCACCAGCT ATGATGGCTGCAGGCCCCCGGA
                                                 -26


 T  S  L  L  L  A  F  T  L  L  C  L  P  W  T  Q  V  V  G  A  F
CCTCCCTGCTCCTGGCTTTCACCCTGCTCTGCCTGCCCTGGACTCAGGTGGTGGGCGCCTTC
                                                             +1


  P  A  M  S  L  S  G  L  F  A  N  A  V  L  R  A  Q  H  L  H  Q
CCAGCCATGTCCTTGTCCGGCCTGTTTGCCAACGCTGTGCTCCGGGCTCAGCACCTGCATCA


   L  A  A  D  T  F  K  E  F  E  R  T  Y  I  P  E  G  Q  R  Y
ACTGGCTGCTGACACCTTCAAAGAGTTTGAGCGCACCTACATCCCGGAGGGACAGAGATACT


 S  I  Q  N  T  Q  V  A  F  C  F  S  E  T  I  P  A  P  T  G  K
CCATCCAGAACACCCAGGTTGCCTTCTGCTTCTCCGAAACCATCCCAGCCCCCACGGGCAAG


  N  E  A  Q  Q  K  S  D  L  E  L  L  R  I  S  L  L  L  I  Q  S
AATGAGGCCCAGCAGAAATCAGACTTGGAGCTGCTTCGCATCTCACTGCTCCTTATCCAGTC


   W  L  G  P  L  Q  F  L  S  R  V  F  T  N  S  L  V ·F  G  T
GTGGCTTGGGCCCCTGCAGTTCCTCAGCAGAGTCTTCACCAACAGCCTGGTGTTTGGCACCT


 S  D  R  V  Y  E  K  L  K  D  L  E  E  G  I  L  A  L  M  R  E
CGGACCGTGTCTATGAGAAGCTGAAGGACCTGGAGGAAGGCATCCTGGCCCTGATGCGGGAG


  L  E  D  V  T  P  R  A  G  Q  I  L  K  Q  T  Y  D  K  F  D  T
CTGGAAGATGTTACCCCCCGGGCTGGGCAGATCCTCAAGCAGACCTATGACAAATTTGACAC


   N  M  R  S  D  D  A  L  L  K  N  Y  G  L  L  S  C  F  R  K
AAACATGCGCAGTGATGATGCGCTGCTCAAGAACTACGGTCTGCTCTCCTGCTTCCGGAAGG


 D  L  H  K̄  T  Ē  T  Y  L  R  V  M  K  C  R  R  F  G  E  A  S
ACCTGCACAAGACGGAGACGTACCTGAGGGTCATGAAGTGTCGCCGCTTCGGGGAGGCCAGC


 C  A  F
TGCGCCTTCTAGTTGCCAGCCATCTGTTGTTACCCCTCCCC     3'
     191
```

Nucleotide sequence and corresponding amino acid
sequence of oGH12 cDNA. The term +1 refers to the
first amino acid (A:alanine) at the N-terminal end
of the mature hormone;

## FIG 1

$P_R$ c1857

Bgl II

Apa I

ori    ori    par

pMG939

Pvu II

Pst I

EcoRI    T7 term

Restrict with
EcoRI / Bgl II

phosphatase

LMT purify 6·5 kb
pMG 197 vector fragment

EcoRI ———————— Bgl II
6·5kb
pMG 197

5' ——55mer—— 3'
3' ——47mer—— 5'

Kinase 55-mer

Anneal $^{32}$P-55mer
to 47-mer

Bgl II  P————  HgiA1
55 bpGH
oligo

HgiA1    HgiA1

HgiA1
EcoRI    pOGH12
HgiA1
HgiA1
oGHcDNA    HgiA1
EcoRI

Restrict with
EcoRI / HgiA1

LMT Purify 650 bp
oGH cDNA fragment

HgiA1  P————  EcoR1
650bp    P
oGH cDNA

ligate
(pMGoGH)

Transform into E.coli DH1

Screen transformations with
$^{32}$P-55mer oGHcDNA
$^{32}$P-17mer oGHcDNA

Grow up positives
Induce cultures

DNA
EcoRI / Bgl II
650bp insert

pMGoGH2

Transform pMGoGH2
into E.coli IB 392

Screen with $^{32}$P-55mer

Grow up and
screen positives

Coomassie
stain

pMGoGH2.6

Western blot

pMGoGH2.6

Protein
SDS-PAGE

Coomassie
stain

Western Blot

pMGoGH2

ASSEMBLY OF pMGoGH1
EXPRESSION VECTOR

FIG 2

Neu eingereicht / Newly filed
Nouvellement déposé

$\lambda$PR        c1857

Col-E1 ori                                      ori(pSC101)

⊖

trp O/P                    pMGoGH1

BglII

HgiA1                                          T7 term

EcoR1

FIG    3

EP 0 363 063 A2

15 bases missing
from pMGoGH2

Bgl II                                                                    HgiA1

AAGTTCACGTAAAAGGGTATCGATAGATCTATGGCTTTTCCAGCTATGTCTCTTTCTGGTCTGTTCGCTAACGCTGTGCTCCGGGCT

pMGoGH1 5'coding sequence

pMGoGH2 5'coding sequence

FIG   4

Nucleotide   sequence   of   5'coding   region   and   flanking   vector   sequence   of
pMGoGH1    and   pMGoGH2   expression   plasmids.